# EUROPEAN PATENT APPLICATION

(11) **EP 2 759 539 A1**
(43) Date of publication of application: **30.07.2014**
(21) Application number: 14150429.0
(22) Date of filing: 08.01.2014
(51) Int. Cl.: C07D 251/70, A61K 8/49, A61Q 17/04

(54) **Triazine oligomers as photostabilising agents**

(30) Priority: 25.01.2013 IT MI20130119
(71) Applicant: 3V SIGMA S.p.A, 20121 Milano (IT)
(72) Inventor: Fabbi, Massimo, 24121 BERGAMO (IT); Seccomandi, Carlo, 24121 BERGAMO (IT); Bemporad, Luca, 24121 BERGAMO (IT); Balestra, Ivan, 24121 BERGAMO (IT)
(74) Representative: Minoja, Fabrizio

(57) **Abstract**

Disclosed are oligomeric compounds obtained by condensation of the compound of formula (I) with itself 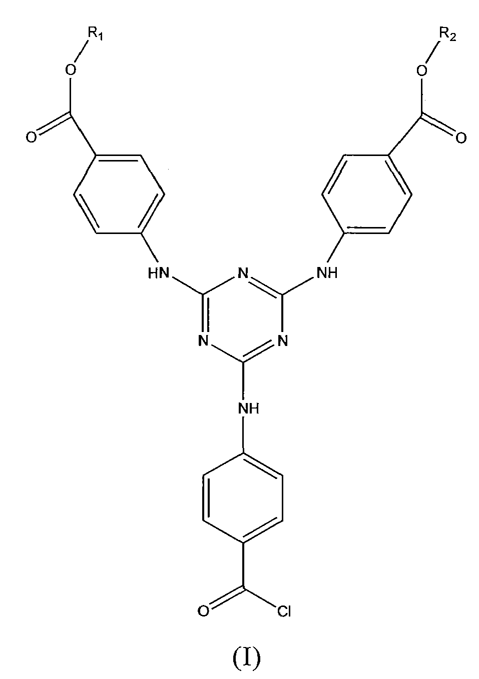 wherein R₁ and R₂ are C₁-C₂₂ alkyl, isoalkyl or cycloalkyl groups.

## Description

The present invention relates to s-triazine oligomer derivatives, their preparation process and their use as light stabilisers.

### Prior art

Ultraviolet solar radiation has a damaging effect on the skin tissue, and causes the degradation of polymers. By using particular compounds, called sunscreens, which absorb the UV part of solar radiation, harmful effects and aging of the skin and polymer materials can be prevented, or at least slowed.

Numerous substances have been studied and tested as protective agents, and a great deal of patent literature now exists relating to compounds belonging to various chemical classes that absorb in the ultraviolet region, particularly radiation between 290 and 320 nm, called UV-B, which is very harmful.

Relatively few of these compounds have proved suitable for practical application. They include p-methoxycinnamic acid and p-dimethylaminobenzoic acid esters, benzotriazoles and hydroxybenzophenones.

A drawback shared by all these compounds is their low ability to absorb radiation between 290 and 320 nm, which means that comparatively large amounts are required to obtain the optimum photoprotective effect.

An excellent UV-B absorber should have the following characteristics:
1) High specific extinction at 290-320 nm allowing the use of low doses, resulting in cost savings and minimal toxicological risk
2) Light stability
3) Heat stability
4) Oxidation stability
5) Stability to different pHs
6) Good solubility in the basic substances commonly used for dermatological formulations
7) Negligible toxicity
8) Colour and odour compatible with the intended applications
9) High molecular weight, which reduces the probability of absorption by the skin and increases toxicological safety
10) Compatibility with the different substances generally used in dermatological formulations.

DE 3206398 discloses s-triazine derivatives obtained by reacting trichlorotriazine with p-amino-benzoic acid esters, which absorb intensely in the UV-B region. Unfortunately, the solubility of these compounds in the solvents generally used to formulate sun creams is very low, which makes their practical use problematic and very difficult, especially when the percentage of photoprotector in the composition must be increased to prepare formulations with a high sun protection factor.

IT 1255729 describes s-triazine derivatives obtained by reacting trichlorotriazine with p-amino-benzoic acid esters or amides with high specific extinction and improved solubility in solvents.

The sun protection factor (SPF) is a measurement of the photoprotective power of a sunscreen or a cosmetic formulation containing one or more sunscreens.

It is directly correlated with specific extinction, and therefore also with the amount of photoprotector present in the cosmetic preparation.

### Description of the invention

It has now been found that oligomers obtainable by polycondensation of s-triazine monomers having a reactive group of formula -COC1 not only absorb very intensely in the UV-B region, but also possess excellent solubility in the solvents most commonly used as ingredients in sunscreen formulations, although they have a very high molecular weight, and are therefore more suitable in toxicological terms since they are less likely to be absorbed by the skin tissue.

The invention also relates to the use of said compounds as sunscreens and photostabilisers, due to their ability to perform a surprising skin protection action against the harmful component of solar radiation.

The compounds according to the invention can also be usefully employed in the photostabilisation of synthetic polymers to prevent photodegradation and deterioration.

In addition to high absorption in the UV-B region and excellent solubility, the oligomers according to the invention also possess other advantageous characteristics, such as heat stability and non-toxicity, associated with their very high molecular weight.

### Detailed description of the invention

The invention relates to compounds obtained by polycondensation of the monomer of formula (I) on itself: 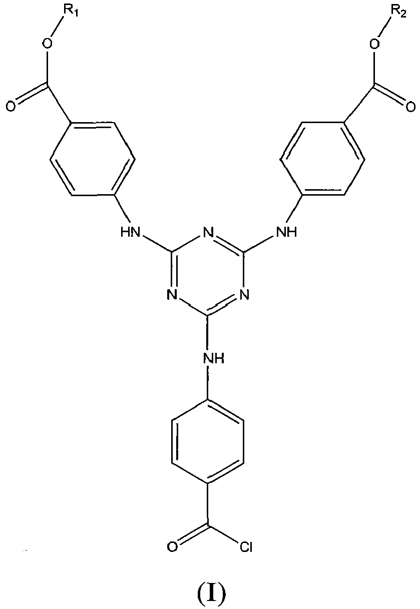 wherein R₁ and R₂ are C₁-C₂₂ alkyl, isoalkyl or cycloalkyl groups.

The -COCl group of the molecule is able to react under certain conditions with each of the 3 -NH- groups of another identical molecule or of the dimers and oligomers that can be produced by this reaction.

The compound of formula (I) is therefore able to generate branched but not crosslinked oligomers and polymers by nonlinear polymerisation according to the model described by P.J. Flory in Principles of Polymer Chemistry, Cornell University Press, 1953, pp. 361-398.

On the basis of these models, the monomer of formula (I) can be conceptually illustrated as a system: 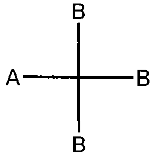 wherein the A group is the -COCl group and the three B groups are the -NH- groups.

The A and B groups can react with each other, but not with themselves.

The reaction between the A and B groups, namely between a -COCI group and an -NH- group, takes place as follows: 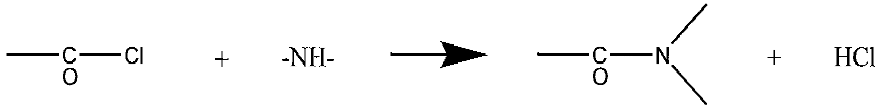 with release of hydrochloric acid.

From the logical standpoint the oligomeric structures can be progressively represented as follows:

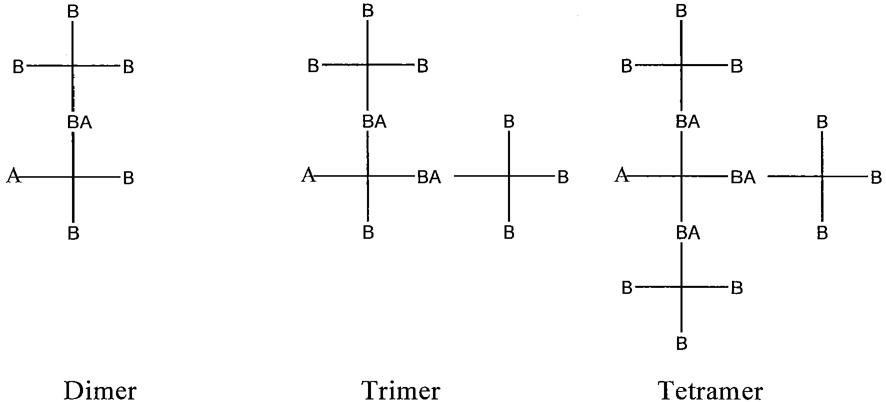

All these structures are characterised in that, as the reaction progresses, only one reactive A (-COCl) group always remains, whereas the B (-NH-) groups multiply.

In the monomer of formula (I), the B groups are not at equivalent positions to the adjacent groups in the molecule.

The monomer of formula (I) can be better conceptually illustrated as follows: 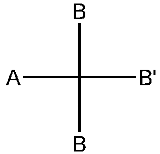 if R₁ = R₂ or with the structure: 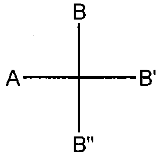 if R₁ is different from R₂
where the A group can react with the B, B' and B" groups but not with itself, and the B, B', B" groups can react with A, but not with each other.

As the reaction and the degree of polymerisation proceeds, numerous oligomers and positional isomers can therefore be generated.

All these structures can be reacted in turn according to well-known synthesis techniques, in particular with alcohols or amines, so as to convert the A group or acid chloride to the corresponding ester or amide group. The preferred alcohols are primary and secondary aliphatic alcohols, and in particular, C₁-C₂₂ straight, cyclic or branched alkyl alcohols. Examples of preferred alcohols are methanol, ethanol, n-propanol, isopropanol, tert-butanol, n-octanol, 2-ethylhexanol, dodecanol and cyclohexanol. The preferred amines are primary or secondary aliphatic amines and, in particular, straight, cyclic or branched C₁-C₂₂ alkyl amines. Examples of preferred amines are methylamine, dimethylamine, ethylamine, diethylamine, propylamine, n-butylamine, sec-butylamine, tert-butylamine, 2-ethylhexylamine, tert-octylamine and cyclohexylamine. An even more preferred alcohol is 2-ethylhexanol, and an even more preferred amine is tert-butyl amine.

To exemplify the matters described above on one of the simplest structures, such as the dimer, and taking account of the possible configurational isomers, its structure can be represented by formula (II): 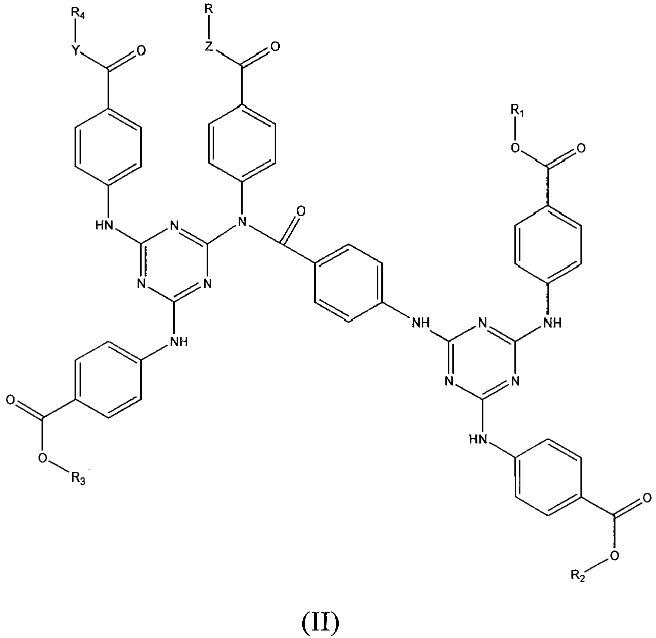 wherein:
Y and Z are -O- or -NH- but are not both -NH-, and
when Y and Z are both -O-, R, R₁, R₂, R₃ and R₄ are the same as one another and are hydrogen, C₁-C₂₂ straight or branched alkyl or cycloalkyl groups;
when Y is -O- and Z is -NH-, R is a C₁-C₂₂ straight or branched alkyl or cycloalkyl group which can be the same or different from groups R₁, R₂, R₃ and R₄ which are hydrogen, C₁-C₂₂ straight or branched alkyl or cycloalkyl groups which are the same as one another;
when Y is -NH- and Z is -O-, R₄ is a C₁-C₂₂ straight or branched alkyl or cycloalkyl group which can be the same or different from groups R, R₁, R₂ and R₃, which are hydrogen, C₁-C₂₂ straight or branched alkyl or cycloalkyl groups which are the same as one another.

Groups R, R₁, R₂, R₃ and R₄ are preferably hydrogen, methyl, ethyl, propyl, isopropyl, butyl, isobutyl, tert-butyl, octyl and 2-ethylhexyl.

Even more preferably,
when Y and Z are both -O-, R, R₁, R₂, R₃ and R₄ are the 2-ethylhexyl group;
when Y is -O- and Z is -NH-, R is the tert-butyl group, while R₁, R₂, R₃ and R₄ are the 2-ethylhexyl group;
when Y is -NH- and Z is -O-, R₄ is the tert-butyl group, while R, R₁, R₂ and R₃ are the 2-ethylhexyl group.

It should be emphasised that the dimers, oligomers and polymers according to the invention are branched but not crosslinked, and are therefore soluble in solvents.

The dimers, oligomers and polymers according to the invention can be prepared by self-condensation of the compound of general formula (I) under acid, neutral or basic conditions and in the presence or absence of suitable inert solvents at temperatures between 0 and 200°C, to obtain an oligomer species such as acyl chloride which is then suitably reacted with amines or alcohols according to well-known conventional techniques.

The dimers, oligomers and polymers according to the invention can also be prepared by self-condensation of the compound of general formula (I) in the presence of triazine compounds with a similar structure such as diethylhexyl butamido triazone and ethylhexyl triazone.

The self-condensation reaction of the compound of formula (I) can be effected in the presence of acidity acceptor such as inorganic bases like oxides, hydroxides, bicarbonates and carbonates of alkali and alkaline earth metals, preferably of sodium, potassium and calcium. Acidity acceptors such as tertiary amines can preferably be used, for example trialkylamines such as trimethylamine and triethylamine.

The solvents in which the preparation of the oligomers according to the invention can be performed need not necessarily be able to dissolve the compounds of formula (I) or the oligomers produced. However, they must not interact under the reaction conditions with the compounds of formula (I) and the corresponding oligomers. In this respect they must be inert. Examples of solvents which can be used are saturated straight and branched hydrocarbons such as hexane, cyclohexane, methylcyclohexane, heptane, octane, isooctane, decane, petrol and dearomatised white spirit, aromatic hydrocarbons such as benzene, toluene, xylenes, ethylbenzene, petrol and white spirit, also containing aromatic hydrocarbons, ketones such as acetone, methyl ethyl ketone, methyl isobutyl ketone, diisobutyl ketone, ethers such as tetrahydrofuran and dioxane, esters such as ethyl acetate and butyl acetate, and nitriles such as acetonitrile and benzonitrile.

The operating temperatures are between 0°C and 200°C, and preferably between 40 and 150°C. The pressures can range between 0 and 50 bar, preferably between 0 and 5 bar.

The compounds of formula I disclosed in Italian patent application MIA000644 of 18.4.2012 can be prepared by reacting cyanuryl chloride or bromide with one p-amino-benzoic acid equivalent, and subsequently reacting the compound obtained with two equivalents of a suitable para-aminobenzoic acid ester and chlorinating with agents such as thionyl chloride, sulphuryl chloride, phosphorus trichloride, phosphorus pentachloride, phosphorus oxychloride, oxalyl chloride, phosgene, or mesyl or tosyl halides.

Alternatively, the order of the first steps can be reversed, reacting the cyanuryl halide first with two equivalents of the p-aminobenzoate esters and then with one equivalent of p-amino-benzoic acid.

The reaction between cyanuryl halide and p-amino-benzoic acid is effected in a solvent, in the presence of a base which can also be added at a second stage, to promote the formation of the product of monosubstitution.

The oligomer derivatives according to the invention can be advantageously introduced into formulas for cosmetics, either as the only sunscreen or in combination with other known sunscreens.

These formulations are a second subject of the invention. Said formulations will preferably contain one or more conventional UVA and UVB sunscreens such as those listed in Annex VII to the European Cosmetics Directive (76/768/EEC). Even more preferably, the formulations may contain, in addition to the oligomer derivatives according to the invention, one or more sunscreens selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, ethylhexyl triazone, 4-(tert-butyl)-4'-methoxy-dibenzoylmethane, 2-cyano-3,3-diphenylacrylic acid 2-ethylhexyl ester, bis-ethylhexyloxyphenol-methoxyphenyl-triazine, methylene-bis-benzotriazolyl-tetramethyl-butylphenol, benzoic acid 2-(4-diethylamino-2-hydroxybenzoyl)-hexyl ester, titanium dioxide, zinc oxide.

The invention will be now be described in greater detail by means of the following examples and preparations.

### Preparation 1: synthesis of 4-((4,6-bischloro-1,3,5-triazin-2-yl)amino)benzoic acid

75.0 g of cyanuryl chloride, 37.3 g of sodium bicarbonate and 304 g of anhydrous acetone, precooled to -10°C, were loaded into a 2-litre flask fitted with a stirrer, thermometer, condenser and dropping funnel.

A solution, precooled to -10°C, consisting of 54.7 g of p-aminobenzoic acid and 523 g of anhydrous acetone, was added in 45 min, under stirring at -10°C.

After 60 minute stirring at -10°C, 100 g of demineralised water, precooled to 0-2°C, was added in approx. 15 minutes. After two more hours of completion at -10°C the product, in the form of a white solid in suspension, was isolated by filtration under vacuum. The wet filtration cake was then washed in sequence, first with aqueous acetone and then with anhydrous acetone. The wet cake was dried in the oven under vacuum to obtain 139 g of fine white powder, consisting of a mixture of the desired product and inorganic salts. The powder was analysed, determining an active chlorine content of 19.8 %w as the difference between total chlorine (29.4 p%) and free chlorides (9.6 p%). The product was also characterised by UPLC-MS chromatography.

### Preparation 2: synthesis of 4-(4,6-bis(4-((2-ethylhexyloxy)carbonyl)phenylamino)-1,3,5-triazin-2-ylamino)benzoic] acid

568 g of anhydrous xylene and 139.0 g of the product prepared in example 1 were loaded into a 2-litre flask fitted with a stirrer, thermometer, dropping funnel and condenser.

645 g of a 30% xylene solution of 2-ethyl hexyl 4-aminobenzoate was added in 30 minutes to the mixture, under stirring at 90°C. When the addition had been completed, the mixture was maintained at 90°C for 15 min and then heated to 125°C in 60 minutes, obtaining a whitish slurry. The mixture was maintained under stirring at 125°C for 3 hours, during which time a gradual reduction in the development of hydrochloric acid and an increase in the fluidity of the mixture was observed. After cooling to 80-90°C, 280 g of 15% aqueous sodium carbonate was added cautiously. After 30 minute mixing at 70-80°C, stirring was interrupted and the underlying alkaline aqueous phase was discharged. After two further aqueous washings, the residual water was removed by azeotropic distillation under atmospheric pressure, followed by distillation of xylene to concentrate the solution.

680 g of whitish dispersion was obtained, containing approx. 275 g of the desired product, which was characterised by UPLC-MS. The dispersion "as is" was used for the subsequent synthesis steps.

### Preparation 3: synthesis of 4-(4,6-bis(4-((2-ethylhexyloxy)-carbonyl)phenylamino)-1,3,5-triazin-2-ylamino)benzoyl chloride of formula I

62.4 g of thionyl chloride, 31.2 g of anhydrous xylene and 0.22 g of dimethylformamide were loaded into a 1-litre flask fitted with a stirrer, thermometer, condenser and dropping funnel. 220 g of the final dispersion obtained in example 2 was fed in 3 hours into the well-stirred mixture, maintained at 70°C.

The hydrochloric acid and sulphur trioxide released during dripping were removed by bubbling in a sodium hydroxide aqueous solution. After completion of the addition, the mixture was stirred at 70°C for a further 2 hours. The excess thionyl chloride was then removed by distillation under vacuum, and the excess xylene as distillation tail. 235 g of xylene mixture, containing approx. 89 g of the desired acyl chloride, remained in the flask. The mixture was directly used for the successive functionalisation reactions, to give a variety of triazine derivatives. A sample of acyl chloride was isolated for characterisation by complete removal of the solvent. The acyl chloride was reacted with an excess of methanol to obtain the corresponding methyl ester, the structure of which was confirmed by IR, NMR and UPLC-MS analysis.

### Example 1 - Intermediate - self-condensation of the compound of formula (I) wherein R₁=R₂=-2-ethylhexyl - Preparation of oligomeric compound 1 according to the invention

500 g of a dispersion of the compound of formula (I), wherein R₁ and R₂ are the 2-ethylhexyl group with 38%w of active ingredient in xylene, was mixed with a solution consisting of 100 g of triethylamine in 100 g of xylene and heated to 110°C under stirring for 6 hours, to obtain 700 g of a dispersion of intermediate. A sample of said intermediate was refluxed with a methanol excess to make the oligomer analysable. GPC analysis in THF of the methyl ester derivative demonstrated that the intermediate had the following composition by weight: compound of formula (I) wherein R₁ = R₂ = -2-ethylhexyl 23.5%w, dimer 18.9%w and oligomers 57.6%w.

### Example 2 - Preparation of oligomeric compound 2 according to the invention

50 g of 2-ethylhexanol was added to 230 g of the intermediate dispersion of example 1. The mixture was reacted at 100°C for 2 hours. After cooling, the mixture was washed at 80°C with 80 g of a 9% w/w sodium carbonate aqueous solution, and then washed twice, each time with 50 g of water. The organic phase was diluted with 1300 g of dearomatised white spirit with a boiling point between 120°C and 150°C. The dispersion obtained was filtered, and the solid washed twice with water and resuspended in white spirit. The solid oligomer product was recovered by filtration and drying. The specific extinction value E¹₁ was 564. The softening point, determined with the DSC method, was between 65°C and 80°C.

### Example 3 - Preparation of oligomeric compound 3 according to the present invention

230 g of the intermediate dispersion of example 1 was added to a solution of 46 g of tert-butylamine in 46 g of xylene. The mixture was heated and refluxed at 90°C for 2 hours.

90 g of a 9% w/w sodium carbonate aqueous solution was added. The reaction mixture was dried by distillation of the excess amine, water and part of the xylene. The anhydrous dispersion was filtered. The clear xylene solution was dropped in n-heptane to obtain a precipitate. The solid oligomer product was recovered by filtration, washing with n-heptane and water and drying. The specific extinction value E¹₁ was 1186.

### Example 4 - Characterisation of the dimers of oligomeric compound 3

The dimers of the oligomer mixture of compound 3 were separated from the oligomers and the corresponding monomer by chromatography techniques. In particular, the thin-layer reverse-phase chromatography technique proved effective, or solid-phase extraction also in reverse phase (apolar stationary phase). The mixture was dissolved in methanol and applied to the chromatography column. Elution with methanol alone separated a fraction almost exclusively containing the monomer; when dichloromethane was added to the methanol in increasing proportions, a dimer-rich fraction was separated, followed by an oligomer-rich fraction.

The dimer-enriched fraction was characterised by NMR techniques (1H and 13C NMR), Size Exclusion Chromatography (SEC), HPLC-UV and UPLC-MS, to effect a complete structural characterisation.

Starting with the compound of formula (I), it can react with the three NH groups of a second identical structure to generate two different dimer structures as two NHs are chemically equivalent. Assuming that the three NH groups have the same reactivity, two dimer structures should be obtained, in the approximate ratio of 2:1.

In particular, the two structures consist of the compound of formula (II) where,

when Y is -NH- and Z is -O-, R₄ is the tert-butyl group while R, R₁, R₂ and R₃ are the 2-ethylhexyl group
and,
when Y is -O- and Z is-NH-, R is the tert-butyl group while R₁, R₂, R₃ and R₄ are the 2-ethylhexyl group.

This is confirmed by the experimental findings; in particular:

The HPLC-UV chromatogram of the fraction shows two chromatographic peaks with an elution time of 34.4 and 35.0 minutes with an area ratio of 28.5 and 61.8% respectively. The visible UV spectrum is identical to that of the monomer with an absorption peak at 309 nm. These peaks are clearly differentiatable from the monomer which, if it had been present, would have had an elution time of 28.4 minutes.

The UPLC-MS chromatogram allows the same mass peak of 1458 daltons to be attributed to both; in fact, the positive-ion ionisation shows an M+H⁺ peak at 1459 daltons, and the negative-ion ionisation shows a corresponding (M-H)⁻ peak at 1457 daltons.

The SEC chromatogram, separating according to molecular weight, only shows one peak (with a hydrodynamic volume of 1548 daltons by reference to polystyrene), further confirming that both dimer structures have the same molecular weight.

The ¹H NMR spectrum confirms the presence of two dimer structures in the weight ratio of 2:1. In particular the following are diagnostic:
1) the two singlets at 6.01 and 5.88 ppm, attributable to the NH protons bonded to the alkyl structure (the NH of the corresponding monomer resonates at 5.91 ppm),
2) the two CH2-O multiplets of the -2ethylsilica chain, again in the ratio of 2:1 in the 4.15-4.35 ppm region.

### Example 5

The mixtures of dimers and oligomers of compounds 2 and 3 were also tested according to their ability to perform a photoprotective action. Said mixtures were added to standard cosmetic formulas (formulas shown in table 1) to evaluate the value of SPF (sun protection factor) with a Labsphere UV-2000S instrument, in the UV-visible region from 290 to 400 nm. For the experimental measurement of SPF, the cosmetic formula was applied to Transpore tape (3M Inc.) at the concentration of 2.0 mg/cm². 3 tapes were prepared for each formula, 12 readings per tape being conducted; readings with a covariance >10% above the average were rejected. The SPF data are set out in table 2.

**Table 1**

| **Phase** | **Ingredient** | **INCI name** | **Formula 1** | **Formula 2** |
|---|---|---|---|---|
| **A1** | Water | water | 72,5 | 72,5 |
| **A1** | Propylene Glycol | propylene glycol | 1 | 1 |
| **A2** | Satiaxane CX91 | xanthan gum | 0,6 | 0,6 |
| **A2** | Ultrez 10 | Carbomer | 0,15 | 0,15 |
| **A2** | Disodium EDTA | disodium EDTA | 0,08 | 0,08 |
| **B1** | Lanette 16 | cetyl alcohol | 1 | 1 |
| **B1** | Tego alkanol S21P | steareth-21 | 2,5 | 2,5 |
| **B1** | Tego alkanol S2P | steareth-2 | 3 | 3 |
| **B1** | Cetiol CC | dicaprylyl carbonate | 6,5 | 6,5 |
| **B1** | Tegosoft DC | decyl cocoate | 6,5 | 6,5 |
| **B2** | Oligomeric compound 2 | | 1 | |
| **B2** | Oligomeric compound 3 | | | 1 |
| **C** | TEA | TEA | 0,225 | 0,225 |
| **D** | Dow Corning 245 | cyclomethicone | 2 | 2 |
| **D** | Microcare PMS | phenoxyethanol and paraben | 1 | 1 |

Preparation: Phase B1 was heated to 70°-75°C under stirring, and B2 was then added. A1 was heated separately to 70°-75°C, adding phase A2 and homogenising with a turboemulsifier. Maintaining the temperature at 70°-75°C, B1+B2 was poured into A1, and homogenised with a turboemulsifier. After adding C, the formulation was cooled to 40°C, and phase D was then added, again under stirring.

**Table 2**

| **Formula** | **Mean SPF** | **Std. deviation SPF** | **UVA:UVB ratio** | **Critical wavelength** |
|---|---|---|---|---|
| **1** | 2.32 | 0.39 | 0.15 | 333.0 |
| **2** | 3.12 | 0.78 | 0.13 | 330.3 |

## Claims

1. Oligomeric compounds obtained by self-condensation of the compound of formula (I) 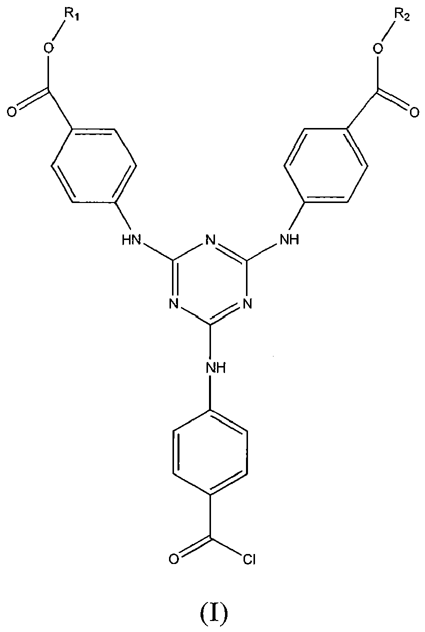 in which R₁ and R₂ are C₁-C₂₂ alkyl, isoalkyl or cycloalkyl groups.

2. Compounds according to claim 1 comprising two residues of compounds of formula I.

3. Compounds according to claims 1 or 2 wherein the -COCl residues are derivatised with alcohols or amines.

4. Compounds according to claim 1, 2 or 3 wherein R₁=R₂=-2-ethylhexyl.

5. Compounds according to claim 3 wherein the amine is tert-butylamine.

6. Compounds according to claim 3 wherein the alcohol is 2-ethylhexanol.

7. Compounds of formula (II) 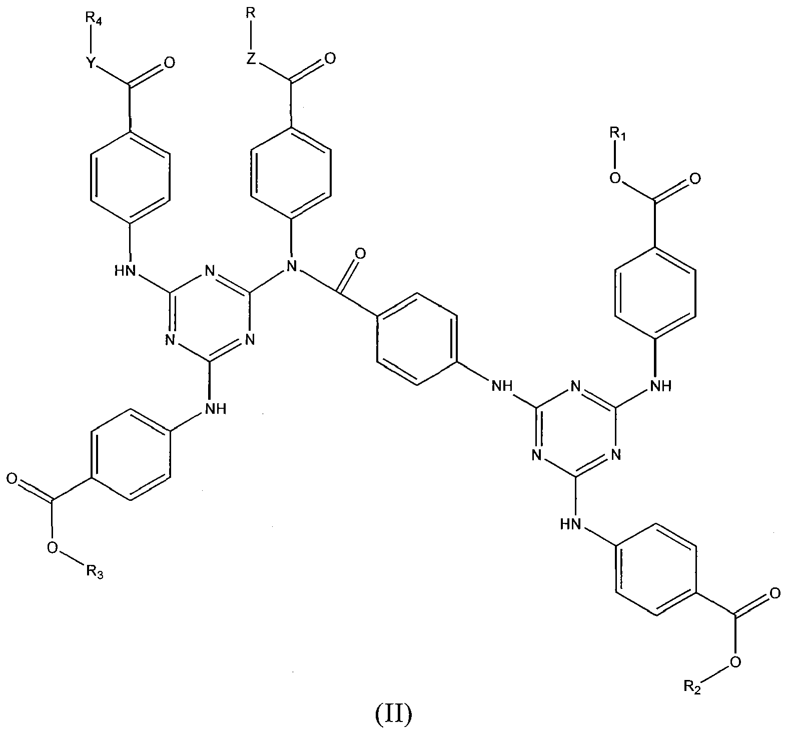 wherein:
Y and Z are -O- or -NH- with the proviso that they are not both -NH-
and
when Y and Z are both -O-, R, R₁, R₂, R₃ and R₄ are the same and are hydrogen, C₁-C₂₂ straight or branched alkyl or cycloalkyl groups;
when Y is -O- and Z is-NH-, R is a C₁-C₂₂ straight or branched alkyl or cycloalkyl group which can be the same or different from R₁, R₂, R₃ or R₄ groups which are the same and are selected from hydrogen, C₁-C₂₂ straight or branched alkyl or cycloalkyl groups;
when Y is -NH- and Z is -O-, R₄ is a C₁-C₂₂ straight or branched alkyl or cycloalkyl group which can be the same or different from R, R₁, R₂ and R₃ groups which are the same and are selected from hydrogen, C₁-C₂₂ straight or branched alkyl or cycloalkyl groups.

8. Compounds of formula (II) according to claim 7 wherein:
when Y and Z are both -O-, R, R₁, R₂, R₃ and R₄ are 2-ethylhexyl
when Y is -O- and Z is-NH-, R is tert-butyl while R₁, R₂, R₃ and R₄ are 2-ethylhexyl;
when Y is -NH- and Z is -O-, R₄ is tert-butyl while R, R₁, R₂ and R₃ are 2-ethylhexyl.

9. Cosmetic compositions containing the compounds of claims 1 to 7 alone or in admixture with one or more UVA and UVB sunscreens.

10. Cosmetic compositions according to claim 8 also containing one or more sunscreens selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, ethylhexyl triazone, 4-(tert-butyl)-4'-methoxy-dibenzoylmethane, 2-ethylhexyl 2-cyano-3,3-diphenylacrylate, bis-ethylhexyloxyphenol-methoxyphenyl-triazine, methylene-bis-benzotriazolyl-tetramethylbutyl-phenol, benzoic acid 2-(4-diethylamino-2-hydroxybenzoyl)-hexyl ester, titanium dioxide, zinc oxide.

11. Use of the compounds of claims 1 to 8 as sunscreens.
